# EUROPEAN PATENT SPECIFICATION

(11) **EP 0 843 002 B1**
(45) Date of publication and mention of the grant of the patent: **12.01.2011**
(21) Application number: 97118711.7
(22) Date of filing: 28.10.1997
(51) Int. Cl.: A61K 8/34, A61K 8/39, A61K 8/41, A61K 8/46, A61Q 19/10

(54) **Detergent composition**
Waschmittel
Composition détergente

(30) Priority: 15.11.1996 JP 30525296; 15.11.1996 JP 30525396
(43) Date of publication of application: 20.05.1998
(73) Proprietor: KAO CORPORATION, Chuo-ku, Tokyo (JP)
(72) Inventor: Shonaka, Masafumi, Wakayama-shi, Wakayama (JP); Sonohara, Hiroshi, Wakayama-shi, Wakayama (JP); Hasebe, Keiko, Wakayama-shi, Wakayama (JP); Doi, Yasuhiro, Wakayama-shi, Wakayama (JP)
(74) Representative: HOFFMANN EITLE

(56) References cited:
- EP-A- 0 467 235
- WO-A-96/29983
- PATENT ABSTRACTS OF JAPAN vol. 018, no. 415 (C-1233), 4 August 1994 (1994-08-04) & JP 06 122893 A (TOYO BEAUTY KK), 6 May 1994 (1994-05-06)

## Description

### BACKGROUND OF THE INVENTION

### Field of the Invention:

The present invention relates to detergent compositions, and particularly to detergent compositions which have excellent detergency and foamability and high germicidal effect, and are hence excellent in antidandruffy and antipruritic effects on the scalp, and antipruritic and deodorant effects on the body. Description of the Background Art:

In the conventional detergent compositions, an anionic surfactant is often incorporated with a view toward achieving excellent detergency and foamability.

On the other hand, detergents, particularly, hair and body shampoos, are also required to have an germicidal effect in addition to the detergent effect. Many investigations have been made as to detergents having a germicidal effect, and detergent compositions making use of a cationic germicide as a germicide have been known.

However, the combined use of an anionic surfactant with a cationic germicide has involved a problem that they form a complex, so that not only the detergency is lowered, but also the germicidal effect cannot be sufficiently exhibited.

WO 96/29983 relates to mild aqueous detergent compositions that are based on a mixture comprising anionic surfactants and amphoteric surfactants, such as betaines, which are present in the composition at a level from about 0.75 to 1.25 parts per weight per part by weight of anionic surfactant. The compositions also contain one or a mixture of climbazole and one or more co-therapeutics such as salicylic acid. The combination of mild surfactant system and therapeutic agent serves to prevent or treat mild skin disorders such as scalp itch and scalp irritation when applied to the scalp as a shampoo. Shampoo compositions also preferably contain one or more conditioning agents and suitable suspending agents.

EP 0 467 235 A1 relates to hair conditioning shampoos containing a long chain alkyl sulfate and/or long chain alkyl ether sulfate anionic surfactant and a cationic di-long chain alkyl quaternary ammonium nitrogen-containing compound, which is said to unexpectedly increase the ability of the aqueous shampoo to incorporate water-insoluble conditioning agents, particularly non-volatile silicon materials, and which allows to remove previously applied conditioning agents and other soiled conditioning agents and contaminants from the hair.

### SUMMARY OF THE INVENTION

Accordingly, it is an object of the present invention to provide detergent compositions which have excellent detergency and foamability and also superb germicidal effect, and can hence effectively prevent formation of dandruff and itch on the scalp, and itch on and odor of the body.

In view of the foregoing circumstances, the present inventors have carried out an extensive investigation. As a result, it has been found that when a specific cationic germicide and a specific antibacterial agent are incorporated in specific weight proportions in combination with an anionic surfactant, the germicidal effect of the cationic germicide in anions and the effect of the antibacterial agent are synergistically improved, thus leading to completion of the present invention which relates to detergent compositions excellent in effects such as antidandruffy, antipruritic and deodorant effects.

According to the present invention, there is thus provided a detergent composition comprising the following components (A), (B) and (C):
(A) 5-95 wt.% of an anionic surfactant;
(B) 0.2-5 wt.% of at least one germicide selected from the group consisting of cationic germicides represented by the following general formula (2): 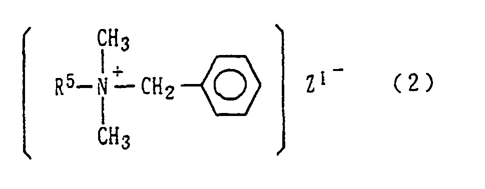 wherein R⁵ is a hydrocarbon group having 8-14 carbon atoms or a group represented by the formula: 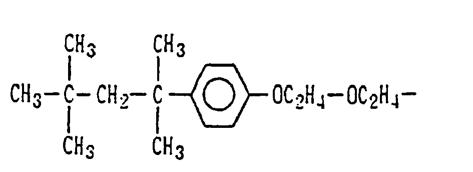 Z¹ is a halogen atom, an anionic residue of an amino acid, fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin;
(C) at least one antibacterial agent selected from the group consisting of triclosan, triclocarban, DMDM hydantoin, piroctone olamine, zinc pyrithione, selenium disulfide, climbazole and 3-methyl-4-(1-methylethyl)phenol (i.e., isopropyl cresol),
   wherein the weight ratio (B)/(C) of the cationic germicide of the component (B) to the antibacterial agent of the component (C) is 0.1 to 25.

The detergent composition according to the present invention has excellent detergency and foamability, and further has high germicidal effect and wide antibacterial spectrum because the antibacterial agent and germicide are used in combination. Therefore, the composition has a sufficient antidandruffy effect on the scalp, and antipruritic and deodorant effects on the body and scalp.

The above and other objects, features and advantages of the present invention will be readily appreciated as the same becomes better understood from the preferred embodiments of the present invention, which will be described subsequently in detail, and from the appended claims.

### DETAILED DESCRIPTION OF THE PREFERRED EMBODIMENTS

No particular limitation is imposed on the anionic surfactant useful in the practice of the present invention. Examples thereof include higher fatty acid salts, polyoxyalkylene alkyl ether carboxylic acids and salts thereof, alkylsulfates, alkylsulfonates, alkylbenzenesulfonates, polyoxyalkylene alkyl ether sulfates, alkylphosphates, polyoxyalkylene alkyl ether phosphates, polyoxyalkylene alkylamide ether carboxylic acids and salts thereof, alkylsulfosuccinates, polyoxyalkylene alkyl ether sulfosuccinates, N-acyl-N-methyltaurinic acid salts, N-acylsarcosinates, α-olefin-sulfonates, acylated isethionates, and acylated glutamic acid and salts thereof. Of these, the higher fatty acid salts, alkylsulfates, polyoxyalkylene alkyl ether sulfates, alkylphosphates, polyoxyalkylene alkylamide ether carboxylates, acylated isethionates, polyoxyalkylene alkyl ether carboxylic acids, salts thereof and polyoxyalkylene alkyl ether sulfosuccinates are more preferred.

Alkylsulfates or polyoxyalkylene alkyl ether sulfates represented by the following general formula (5):

R⁷O-(CH₂CH₂O)ₙ-SO₃M¹ (5)

wherein R⁷ is a linear or branched alkyl or alkenyl group having 8-20 carbon atoms, n is a number of 0-10 on the average, and M¹ is an alkali metal or alkaline earth metal atom, or an ammonium, alkylammonium or alkanolammonium group; and higher fatty acid salts represented by the
following general formula (6):

R⁸COOX (6)

wherein R⁸ is a linear or branched alkyl or alkenyl group having 7-21 carbon atoms, and X is a potassium or sodium atom, or an ammonium or alkanolammonium group, are particularly preferred.

In the general formula (6), R⁸ is particularly preferably a linear or branched alkyl or alkenyl group having 9-17 carbon atoms.

X is preferably a potassium or sodium atom, or an ammonium or triethanolammonium group.

The above-described anionic surfactants may be used either singly or in any combination thereof. The higher fatty acid salt may be used in combination with any other anionic surfactant. In this case, the resultant detergent composition is suitable for use as a body shampoo.

The component (A) is incorporated in a proportion of 5-95 wt.% based on the total weight of the composition. When the composition is provided in the form of liquid, the component (A) is preferably incorporated in a proportion of 5-50 wt.%, particularly, 8-40 wt.%. When the composition is provided in the form of paste, the component (A) is preferably incorporated in a proportion of 10-80 wt.%, particularly, 15-70 wt.%. When the composition is provided in the form of solid, the component (A) is preferably incorporated in a proportion of 60-95 wt.%, particularly, 70-95 wt.%.

The cationic germicide of the component (B) useful in the practice of the present invention is at least one selected from the group consisting of benzalkonium salts represented by the general formula (2).

In the general formula (2), Z¹ is particularly preferably a halogen atom.

Specific examples of the component (B) include benzalkonium chloride, and benzethonium chloride.

Benzalkonium chloride and benzethonium chloride are preferred, with benzalkonium type germicides represented by the general formula (2a); 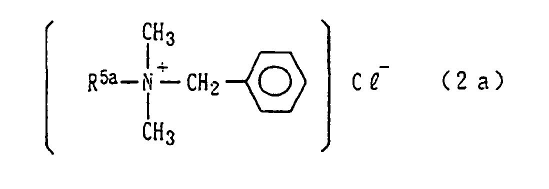 wherein R^{5a} is a linear or branched alkyl or alkenyl group having 8-14 carbon atoms, for example, benzalkonium chloride, being particularly preferred.

The cationic germicides of the component (B) may be used either singly or in any combination thereof, and are incorporated in a proportion of 0.2-5 wt.% based on the total weight of the composition from the viewpoints of germicidal effect and irritation. The germicides are preferably incorporated in a proportion of 0.3-4 wt.%, particularly, 0.4-3 wt.%.

The antibacterial agent of the component (C) useful in the practice of the present invention is at least one selected from the group consisting of triclosan, triclocarban, piroctone olamine, DMDM hydantoin, zinc pyrithione, selenium disulfide, climbazole and 3-methyl-4-(1-methylethyl)phenol (i.e., isopropyl cresol). Of these, triclosan, triclocarban, DMDM hydantoin, zinc pyrithione, piroctone olamine and 3-methyl-4-(1-methyl-ethyl)phenol are preferred.

The proportion of the component (C) is preferably determined in terms of a ratio to the component (B). The weight ratio of the component (B) to the component (C) to be incorporated is preferably within a range of 0.1-25, particularly, 0.1-20 because the synergistic effect of antibacterial activity is obtained.

In the detergent compositions according to the present invention, a metal chelating agent may be added to more enhance the germicidal and antibacterial effects of the compositions.

No particular limitation is imposed on the metal chelating agent used in the present invention so far as it has a capacity to chelate metal ions. However, examples thereof include aminopolycarboxylic acid type chelating agents, aromatic and aliphatic carboxylic acid type chelating agents, amino acid type chelating agents, ether polycarboxylic acid type chelating agents, phosphonic acid type chelating agents such as iminodimethylphosphonic acid (IDP), alkyldiphosphonic acids (ADPAs) and 1-hydroxyethane-1,1-diphosphonic acid (DEQUEST^{™} 2010), hydroxycarboxylic acid type chelating agents, phosphoric acid type chelating agents, chelating agents of the polyelectrolyte (including oligomer electrolyte) type, and dimethylglyoxime (DG). Each of these chelating agents may be in the form of a free acid or a salt such as the sodium, potassium or ammonium salt. The chelating agent may also be in the form of a hydrolyzable ester derivative.

Specific examples of the aminopolycarboxylic acid type chelating agents include:
a) compounds represented by the chemical formula, R⁹(Y)₂;
b) compounds represented by the chemical formula, N(Y)₃;
c) compounds represented by the chemical formula,

   R⁹-N(Y)-CH₂CH₂-N(Y)-R⁹;
d) compounds represented by the chemical formula, R⁹-N(Y)-CH₂CH₂-N(Y)₂;
e) compounds represented by the chemical formula, (Y)₂-R¹⁰-N(Y)₂; and
f) compounds similar to the compounds of e), which contain Y more than 4 groups, for example, a compound represented by the formula:

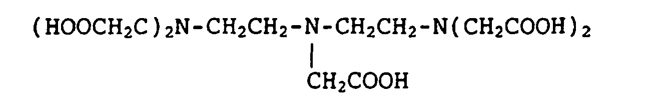

In the above formulae, Y is -CH₂COOH or -CH₂CH₂COOH, R⁹ is a group making up a known chelating agent, such as a hydrogen atom, or an alkyl, hydroxyl or hydroxyalkyl group, and R¹⁰ is a group making up a known chelating agent of this kind, such as an alkylene or cycloalkylene group.

Typical examples of the aminopolycarboxylic acid type chelating agents include ethylenediaminetetraacetic acid (EDTA), cyclohexanediaminetetraacetic acid (CDTA), nitrilotriacetic acid (NTA), iminodiacetic acid (IDA), N-(2-hydroxyethyl)iminodiacetic acid (HIMDA), diethylenetriaminepentaacetic acid (DTPA), N-(2-hydroxyethyl) ethylenediaminetriacetic acid (EDTA-OH) and glycol ether diaminetetraacetic acid (GEDTA) as well as salts thereof.

Examples of the aromatic and aliphatic carboxylic acid type chelating agents used in the present invention include oxalic acid, malonic acid, succinic acid, glutaric acid, adipic acid, itaconic acid, aconitic acid, pyruvic acid, salicylic acid, acetylsalicylic acid, hydroxybenzoic acid, aminobenzoic acid (including anthranilic acid), phthalic acid, trimellitic acid and gallic acid as well as salts, methyl esters and ethyl esters thereof. Examples of the amino acid type chelating agents used in the present invention include glycine, serine, alanine, lysine, cystine, cysteine, ethionine, tyrosine, methionine, and salts and derivatives thereof.

Examples of the ether polycarboxylic acid type chelating agents used in the present invention include diglycolic acid, compounds represented by the following formula: 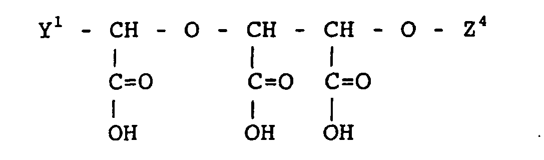 wherein Y¹ is a hydrogen atom, -CH₂COOH or -COOH, and Z⁴ is a hydrogen atom,

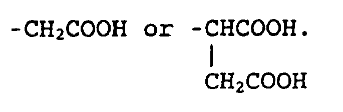

Examples of the hydroxycarboxylic acid type chelating agents used in the present invention include malic acid, citric acid, glycolic acid, gluconic acid, heptonic acid, tartaric acid, lactic acid, and salts thereof. Examples of the phosphoric acid type chelating agents used in the present invention include orthophosphoric acid, pyrophosphoric acid, triphosphoric acid and polyphosphoric acid. Examples of the chelating agents of the polyelectrolyte (including oligomer electrolyte) type used in the present invention include acrylic acid polymers, maleic anhydride polymers, α-hydroxyacrylic acid polymers, itaconic acid polymers, copolymers composed of at least two monomers of these polymers, and epoxysuccinic acid polymers. Further, ascorbic acid, thioglycolic acid, phytic acid, glyoxylic acid and glyoxalic acid as well as salts thereof may also be suitably used as the chelating agents in the present invention.

Preferable examples of the chelating agents include ethylenediaminetetraacetic acid (EDTA), succinic acid, salicylic acid, oxalic acid, lactic acid, fumaric acid, tartaric acid, 1-hydroxyethane-1,1-diphosphonic acid and salts thereof.

The metal chelating agent is incorporated in a proportion of 0.1-10 wt.%, preferably 0.2-5 wt.% based on the total weight of the composition.

To the compositions according to the present invention, antibacterial agents other than the above-described antibacterial agents may be added so far as no detrimental influence is thereby imposed on the effects of the present invention. Examples of such antibacterial agents include those described in "Cosmetic and Drug Preservation Principles and Practice" (John J. Kabara, Marcel Dekker, Inc.).

Further, antiphlogistics may be added so far as no detrimental influence is thereby imposed on the effects of the present invention. Examples of such antiphlogistics include glycyrrhetinic acid, dihydrocholesterin and allantoin.

In the detergent compositions according to the present invention, amphoteric surfactants such as carbobetaine, sulfobetaine and hydroxysulfobetaine, or amine oxide type surfactants, alkanolamide type surfactants, amidoamino acid type surfactants or the like may be further incorporated so far as no detrimental influence is thereby imposed on the effects of the present invention.

Furthermore, silicone derivatives can be incorporated in a proportion of 0.1-5 wt.% to the detergent compositions according to the present invention to give users a pleasant feel of sliding or dryness. No particular limitation is imposed on the silicone derivatives so far as they are those commonly used in the classical detergents and cosmetics. Examples thereof include dimethyl polysiloxane, methylphenyl polysiloxane, polyether-modified silicones, epoxy-modified silicones, alkoxy-modified silicones, amino-modified silicones, fatty acid-modified silicones and fluorine-modified silicones.

Besides, conditioning agents can be incorporated in a proportion of about 0.1-5 wt.% in the detergent compositions according to the present invention to provide conditioning shampoos.

As the conditioning agents, there may be preferably used cationic polymers such as cationic cellulose derivatives, cationic starch, cationic guar gum derivatives, copolymers of a diallyl quaternary ammonium salt and acrylamide, quaternized polyvinyl pyrrolidone derivatives, and polyglycol-amine condensates.

In the detergent compositions according to the present invention, ingredients commonly incorporated in the classical cosmetics, drugs, food and the like, for example, moisturizers such as propylene glycol, glycerol, diethylene glycol monoethyl ether, sorbitol and panthenol; colorants such as dyes and pigments; pearl-like-hue-imparting agents; chitosan derivatives such as hydroxypropylchitosan; various kinds of perfume bases; and besides ingredients described in ENCYCLOPEDIA OF SHAMPOO INGREDIENTS (MICELLE PRESS, 1985), may be incorporated in addition to the above-described components, as needed, so far as no detrimental influence is thereby imposed on the effects of the present invention.

The detergent compositions according to the present invention can be prepared in accordance with a method known *per se* in the art into various forms such as solid, paste, gel and liquid. The detergent compositions are suitable for use as human hair or body shampoos, and can be used for animals such as horses.

The present invention will hereinafter be described in more detail by the following examples. However, the present invention is not limited to these examples. Incidentally, the amounts of individual components to be incorporated in the following examples were expressed on the basis of active ingredients. All designations of "%" as will be used in the following examples mean % by weight. Example 1:

Detergent compositions of their corresponding formulations shown below were prepared to conduct a germicidal test.

| | |
|---|---|
| Coconut fatty acid potassium | 10% |
| salt | |
| Benzalkonium chloride* | 0, 0.2, 0.6, 0.8, |
| | 0.9, 0.95 or 1.0% |
| Triclosan | 0, 0.05, 0.1, 0.2, |
| | 0.4, 0.8 or 1.0% |
| Water | Balance |

| | |
|---|---|
| *: C₁₂/C₁₄ = 50/50 (by weight), the same shall apply hereinafter. | |

In the compositions, benzalkonium chloride and triclosan were incorporated to give a total amount of 1.0%. The germicidal effects of the compositions on *Escherichia coli* are shown in Table 1.

### (Method of germicidal test)

A germ (*Escherichia coli* IFO 3972) to be tested was precultured in advance in an SCD medium (product of Nippon Seiyaku K.K.), and the resultant culture solution was used in the test.

Each (10 ml) of the detergent compositions, which had been diluted to the predetermined concentration (dilution of the composition: by 1, 2, 4, 8, 16, 32, 64, 128, 256 or 512) with sterile water, was inoculated with 0.1 ml (10⁹ to 10¹⁰ cells/ml) of the culture solution. When predetermined periods of time (2.5, 5, 10 and 15 minutes) went on, a portion of the thus-inoculated composition was taken out by a platinum loop and inoculated in a growth medium to conduct culture at 30°C for 3 days, thereby judging whether the germ grew or not.

The results of the germicidal test were expressed in terms of the number of sterilized areas, which is 40 where no growth of the germ was observed over all the diluted concentrations and all the periods of time of the reaction.

**Table 1**

| | Invention product | | | | | Comparative product | |
|---|---|---|---|---|---|---|---|
| | 1 | 2 | 3 | 4 | 5 | 1 | 2 |
| Coconut fatty acid potassium salt (A) | 10 | 10 | 10 | 10 | 10 | 10 | 10 |
| Benzalkonium chloride (B) | 0.95 | 0.9 | 0.8 | 0.6 | 0.2 | 1 | - |
| Triclosan (C) | 0.05 | 0.1 | 0.2 | 0.4 | 0.8 | - | 1 |
| Water | Bal | Bal | Bal | Bal | Bal | Bal | Bal |
| Ratio (B)/(C) of cation to antibacterial agent | 19.00 | 9.00 | 4.00 | 1.50 | 0.25 | - | - |
| Germicidal effect (*E*. *coli*) | 32 | 34 | 33 | 30 | 28 | 20 | 15 |

As apparent from Table 1, it was confirmed that when benzalkonium chloride and triclosan are incorporated in a total proportion of 1.0%, a synergistic effect is recognized compared with the germicidal effect in the case where the individual agents are used by itself.

### Example 2:

Detergent compositions of their corresponding formulations shown in Tables 2 and 3 were used to evaluate their germicidal effects in accordance with the method of the germicidal test described in Example 1. However, *Staphylococcus aureus* IFO 12732 and *Escherichia coli* IFO 3972 were used as test germs to conduct the test.

Further, their foaming and deodorant effects were evaluated in accordance with the following methods and standards. The results are shown collectively in Tables 2 and 3.

### (Evaluation method and standard of deodorant effect)

With respect to each of the detergent compositions, a service test was conducted once a day for 2 weeks with panelists composed of five men and five women.

### (Evaluation method)

The deodorant effect was evaluated by an expert valuer as to whether the panelists had a body smell or not upon elapsed time of 24 hours after the final cleansing and ranked in accordance with the following standard.

| (Evaluation standard) | |
|---|---|
| 5: | Not smelled; |
| 4: | Slightly smelled; |
| 3: | Smelled; |
| 2: | Considerably smelled; and |
| 1: | Strongly smelled. |

The results of the deodorant effect were expressed in terms of the average value of the ten panelists in accordance with the following standard:
⊚: At least 4.0, good deodorant effect;
○ : 3.2-3.9, somewhat good deodorant effect;
Δ : 2.5-3.1, fair deodorant effect; and
×: At most 2.4, poor deodorant effect.

### (Evaluation method and standard of foaming)

At the same time, foaming of each of the detergent compositions when the bodies of the panelists were cleansed with the composition was evaluated in accordance with the following standard.

### (Evaluation standard)

5: Felt that foaming was good;
4: Felt that foaming was somewhat good;
3: Felt that foaming was fair;
2: Felt that foaming was somewhat poor; and
1: Felt that foaming was poor.

The results were expressed in terms of the average value of the ten panelists in accordance with the following standard:
⊚ : At least 4.5, good foaming;
○ : 3.5-4.4, somewhat good foaming;
Δ : 2.5-3.4, fair foaming; and
×: At most 2.4, poor foaming.

**Table 2**

| | | Invention product | | | | | |
|---|---|---|---|---|---|---|---|
| | | 6 | 7 | 8 | 9 | 10 | 11 |
| Component (A) | Sodium laurate | 10.00 | 6.00 | - | 18.00 | 10.00 | - |
| | Coconut fatty acid triethanolamine salt | - | 5.00 | 10.00 | - | - | 11.00 |
| Component (B) | Benzalkonium chloride | 0.50 | 0.50 | 1.00 | 0.50 | 0.75 | 0.50 |
| Component (C) | 3-Methyl-4-(1-methylethyl)phenol | - | - | - | 0.10 | - | 0.05 |
| | Triclosan | - | - | 0.15 | - | - | - |
| | Triclocarban | 0.10 | - | - | - | - | - |
| | DMDM hydantoin | - | 0.10 | - | - | 0.20 | - |
| | Water | Bal | Bal | Bal | Bal | Bal | Bal |
| Ratio (B)/(C) | Ratio of cation to antibacterial agent | 5.00 | 5.00 | 6.67 | 5.00 | 3.75 | 10.00 |
| | Germicidal effect | ○ | ⓞ | ⓞ | ⓞ | ⓞ | ○ |
| | Foaming | ○ | ⓞ | ○ | ⓞ | ○ | ○ |
| | Germicidal effect *(S. aureus)* | 27 | 29 | 29 | 25 | 27 | 25 |
| | Germicidal effect *(E. coli)* | 29 | 30 | 33 | 24 | 25 | 27 |

| | | | | | | | |
|---|---|---|---|---|---|---|---|
| Note) * : C₁₂/C₁₄ =50/50 (by weight). | | | | | | | |

**Table 3**

| | | Comparative product | | | | | |
|---|---|---|---|---|---|---|---|
| | | 3 | 4 | 5 | 6 | 7 | 8 |
| Component (A) | Sodium laurate | 10.00 | 6.00 | - | 18.00 | 10.00 | - |
| | Coconut acid triethanolamine salt | - | 5.00 | 10.00 | - | - | 11.00 |
| Component (B) | Benzalkonium chloride* | - | 0.50 | - | - | - | 0.50 |
| Component (C) | 3-Methyl-4-(1-methylethyl)phenol | - | - | - | 0.10 | - | - |
| | Triclosan | - | - | 0.15 | - | - | - |
| | Triclocarban | 0.10 | - | - | - | - | - |
| | DMDM hydantoin | - | - | - | - | 0.20 | - |
| | Water | Bal | Bal | Bal | Bal | Bal | Bal |
| Ratio (B)/(C) | Ratio of cation to antibacterial agent | - | - | - | - | - | - |
| | Germicidal effect | Δ | Δ | Δ | × | × | Δ |
| | Foaming | ○ | ⓞ | ○ | ⓞ | ○ | ○ |
| | Germicidal effect *(S. aureus)* | 6 | 8 | 6 | 4 | 3 | 6 |
| | Germicidal effect (*E. coli*) | 2 | 10 | 3 | 2 | 3 | 13 |

| | | | | | | | |
|---|---|---|---|---|---|---|---|
| Note) * : C₁₂/C₁₄ =50/ 50 (by weight). | | | | | | | |

As apparent from Tables 2 and 3, it was confirmed that the detergent compositions in which the cationic germicide (B) and the antibacterial agent (C) are used in combination are excellent in foaming and have good germicidal and deodorant effects.

### Example 3:

Detergent compositions of their corresponding formulations shown below were prepared to conduct a germicidal test.

| | |
|---|---|
| Triethanolamine monolauryl | 10% |
| phosphate | |
| Benzalkonium chloride* - | 0, 0.2, 0.6, 0.8, |
| | 0.9, 0.95 or 1.0% |
| Triclocarban | 0, 0.05, 0.1, 0.2, |
| | 0.4, 0.8 or 1.0% |
| Water | Balance |

| | |
|---|---|
| * : C₁₂/C₁₄ = 50/50 (by weight) | |

In the compositions, benzalkonium chloride and triclocarban were incorporated to give a total amount of 1.0%. The germicidal effects of the compositions on *Escherichia coli* are shown in Table 4.

### (Method of germicidal test)

A germ (*Escherichia coli* IFO 3972) to be tested was precultured in advance in an SCD medium (product of Nippon Seiyaku K.K.), and the resultant culture solution was used in the test.

Each (10 ml) of the detergent compositions, which had been diluted to the predetermined concentration (dilution of the composition: by 1, 2, 4, 8, 16, 32, 64, 128, 256 or 512) with sterile water, was inoculated with 0.1 ml (10⁹ to 10¹⁰ cells/ml) of the culture solution. When predetermined periods of time (2.5, 5, 10 and 15 minutes) went on, a portion of the thus-inoculated composition was taken out by a platinum loop and inoculated in a growth medium to conduct culture at 30°C for 3 days, thereby judging whether the germ grew or not.

The results of the germicidal test were expressed in terms of the number of sterilized areas, which is 40 where no growth of the germ was observed over all the diluted concentrations and all the periods of time of the reaction.

**Table 4**

| | Invention product | | | | | Comparative product | |
|---|---|---|---|---|---|---|---|
| | 12 | 13 | 14 | 15 | 16 | 9 | 10 |
| Triethanolamine monolauryl phosphate (A) | 8 | 8 | 8 | 8 | 8 | 8 | 8 |
| Benzalkonium chloride (B) | 0.95 | 0.9 | 0.8 | 0.6 | 0.2 | 1 | - |
| Triclocarban (C) | 0.05 | 0.1 | 0.2 | 0.4 | 0.8 | - | 1 |
| Water | Bal | Bal | Bal | Bal | Bal | Bal | Bal |
| Ratio (B)/(C) of cation to antibacterial agent | 19.00 | 9.00 | 4.00 | 1.50 | 0.25 | - | - |
| Germicidal effect (E. coli) | 31 | 32 | 31 | 30 | 27 | 19 | 15 |

As apparent from Table 4, it was confirmed that when benzalkonium chloride and triclocarban are incorporated in a total proportion of 1.0%, a synergistic effect is recognized compared with the germicidal effect in the case where the individual agents are used by itself.

### Example 4:

Detergent compositions of their corresponding formulations shown in Table 5 were used to evaluate their germicidal effects in accordance with the method of the germicidal test described in Example 3. However, test germs, *Escherichia coli* IFO 3972 and *Staphylococcus aureus* IFO 12732, and *Malassezia furfur* IFO 0656 were precultured in advance in an SCD medium and a Malt-YE-Tween 80 medium, respectively, and the resultant culture solutions were used in the test.

Besides, *E. coli* and *St. aureus* were grown on a SCD medium, while *M. furfur* was grown on a Malt-YE-Tween 80 medium.

Further, their deodorant and antidandruffy effects were evaluated in accordance with the following methods and standards. The results are shown in Table 6.

### (Evaluation method and standard of antidandruffy effect)

With respect to each of the detergent compositions, the antidandruffy effect was evaluated by shampooing hair of panelists composed of five men and five women with the composition once a day for 4 weeks and ranked in accordance with the following standard.

| (Evaluation standard) | |
|---|---|
| 5: | Felt that dandruff was decreased; |
| 4: | Felt that dandruff was somewhat decreased; |
| 3: | Felt that no change was recognized; |
| 2: | Felt that dandruff was somewhat increased; and |
| 1: | Felt that dandruff was increased. |

The results of the antidandruffy effect were expressed in terms of the average value of the ten panelists in accordance with the following standard:
⊚ : At least 4.3, good antidandruffy effect;
○ : 3.6-4.2, somewhat good antidandruffy effect;
Δ : 2.5-3.5, fair antidandruffy effect; and
×: At most 2.4, poor antidandruffy effect.

### (Evaluation method and standard of deodorant effect)

With respect to each of the detergent compositions, a service test was conducted once a day for 2 weeks with panelists composed of five men and five women.

The deodorant effect was evaluated by an expert valuer as to whether the panelists had a body smell or not upon elapsed time of 24 hours after the final cleansing and ranked in accordance with the following standard.

| (Evaluation standard) | |
|---|---|
| 5: | Not smelled; |
| 4: | Slightly smelled; |
| 3: | Smelled; |
| 2: | Considerably smelled; and |
| 1: | Strongly smelled. |

The results of the deodorant effect were expressed in terms of the average value of the ten panelists in accordance with the following standard:
⊚ : At least 4.0, good deodorant effect;
○ : 3.2-3.9, somewhat good deodorant effect;
Δ : 2.5-3.1, fair deodorant effect; and
×: At most 2.4, poor deodorant effect.

**Table 5**

| | | Invention product | | | | | | Comparative product | | | | | |
|---|---|---|---|---|---|---|---|---|---|---|---|---|---|
| | | 17 | 18 | 19 | 20 | 21 | 22 | 11 | 12 | 13 | 14 | 15 | 16 |
| Component (A) | Sodium polyoxyethylene (2) lauryl ether sulfate | 10 | - | - | - | 8 | - | 10 | - | - | - | 8 | - |
| | Sodium polyoxyethylene (3) lauric acid amide ether acetate | - | - | 7 | - | - | 12 | - | - | 7 | - | - | 12 |
| | Sodium monolauryl phosphate | - | 8 | - | 18 | - | - | - | 8 | - | 18 | - | - |
| Component | Benzalkonium chloride* | 0.5 | - | 1 | 0.5 | - | 0.5 | 0.5 | - | - | - | - | 0.5 |
| (B) | Benzethonium chloride | - | 0.5 | - | - | 0.75 | - | - | - | - | - | 0.75 | - |
| Component (C) | 3-Methyl-4-(1-methylethyl)-phenol | - | - | - | 0.075 | - | - | - | - | - | 0.075 | - | - |
| | Triclosan | - | - | 0.1 | - | - | - | - | - | 0.1 | - | - | - |
| | Triclocarban | - | 0.025 | - | - | - | 0.15 | - | 0.025 | - | - | - | - |
| | DMDM hydantoin | - | 0.050 | - | - | - | - | - | 0.050 | - | - | - | - |
| | Pirocton olamine | 0.2 | - | - | - | 0.15 | - | - | - | - | - | - | - |
| | Water | Bal | Bal | Bal | Bal | Bal | Bal | Bal | Bal | Bal | Bal | Bal | Bal |

| | | | | | | | | | | | | | |
|---|---|---|---|---|---|---|---|---|---|---|---|---|---|
| Note) In the table, the figure in ( ) after polyoxyethylene means an average number of moles added. * : C₁₂/C₁₄ =50/50 (by weigh). | | | | | | | | | | | | | |

**Table 6**

| | Invention product | | | | | | Comparative product | | | | | |
|---|---|---|---|---|---|---|---|---|---|---|---|---|
| | 17 | 18 | 19 | 20 | 21 | 22 | 11 | 12 | 13 | 14 | 15 | 16 |
| Ratio (B/C) of cation to antibacterial agent | 2.50 | 6.67 | 10.00 | 6.67 | 5.00 | 3.33 | - | - | - | - | - | - |
| Deodorant effect | ○ | ⊚ | ⊚ | ⊚ | ⊚ | ⊚ | Δ | × | Δ | Δ | Δ | Δ |
| Anti-dandruff effect | ⊚ | ○ | ⊚ | ○ | ⊚ | ○ | Δ | Δ | × | × | Δ | Δ |
| Germicidal effect *(S. aureus)* | 27 | 27 | 29 | 25 | 27 | 25 | 10 | 0 | 6 | 4 | 12 | 6 |
| Germicidal effect *(E. coli)* | 25 | 29 | 31 | 25 | 25 | 26 | 12 | 0 | 2 | 0 | 8 | 7 |
| Germicidal effect *(M. furfur)* | 30 | 26 | 29 | 23 | 28 | 24 | 10 | 4 | 7 | 2 | 8 | 3 |

As apparent from Tables 5 and 6, it was confirmed that when the cationic germicide (B) and the antibacterial agent (C) are used in combination like the invention products, good germicidal, antidandruffy and deodorant effects are recognized due to the synergistic effect thereof.

### Example 5: (Body shampoo)

A body shampoo of a formulation shown below was produced in accordance with a method known *per se* in the art.

| | |
|---|---|
| Coconut fatty acid potassium salt | 21% |
| Lauroyldiethanolamide | 1% |
| Benzalkonium chloride* | 0.5% |
| Triclocarban | 0.1% |
| Disodium succinate | 0.4% |
| Perfume base | 0.5% |
| Purified water | Balance |

The body shampoo thus obtained had excellent detergency and foamability and also high deodorant effect.

### Example 6: (Body shampoo)

A body shampoo of a formulation shown below was produced in accordance with a method known *per se* in the art.

| | |
|---|---|
| Potassium laurate | 16% |
| Potassium palmitate | 2% |
| Sodium polyoxyethylene (2) lauryl | 1.5% |
| ether sulfate | |
| Lauroyldiethanolamide | 1% |
| Benzalkonium chloride* | 1% |
| Triclosan | 0.1% |
| Disodium ethylenediaminetetraacetate | 1.0% |
| Perfume base | 0.5% |
| Purified water | Balance |

The body shampoo thus obtained had excellent detergency and foamability and also high germicidal effect.

### Example 7: (Pasty soap)

A pasty soap of a formulation shown below was produced in accordance with a method known *per se* in the art.

| | |
|---|---|
| Coconut fatty acid sodium salt | 60% |
| Lauroyldiethanolamide | 1% |
| Benzalkonium chloride* | 0.8% |
| Triclocarban | 0.4% |
| Disodium ethylenediaminetetraacetate | 0.8% |
| Perfume base | 0.5% |
| Purified water | Balance |

The pasty soap thus obtained had excellent detergency and foamability and also high deodorant effect.

### Example 8: (Solid soap)

A solid soap of a formulation shown below was produced in accordance with a method known *per se* in the art.

| | |
|---|---|
| Sodium laurate | 85% |
| Coconut fatty acid sodium salt | 8% |
| Lauroyldiethanolamide | 1% |
| Benzethonium chloride | 0.5% |
| Triclosan | 0.1% |
| Triclocarban | 0.1% |
| Disodium ethylenediaminetetraacetate | 0.5% |
| Perfume base | 0.5% |
| Purified water | Balance |

The solid soap thus obtained had excellent detergency and foamability and also high deodorant effect.

### Example 9: (Antidandruffy shampoo)

An antidandruffy shampoo of a formulation shown below was produced in accordance with a method known *per se* in the art.

| | |
|---|---|
| Sodium polyoxyethylene (2) lauryl | 10% |
| ether sulfate | |
| Lauroyldiethanolamide | 1% |
| Lauric acid amide propylbetaine | 3% |
| Benzalkonium chloride | 0.5% |
| Piroctone olamine | 0.2% |
| Disodium succinate | 0.5% |
| Perfume base | 0.5% |
| Purified water | Balance |

The antidandruffy shampoo thus obtained had excellent detergency and foamability and also high antidandruffy effect.

### Example 10 : (Body shampoo)

A body shampoo of a formulation shown below was produced in accordance with a method known *per se* in the art.

| | |
|---|---|
| Sodium polyoxyethylene (3) lauric | 10% |
| acid amide ether acetate | |
| Sodium polyoxyethylene (10) laurylether acetate | 5% |
| Benzalkonium chloride | 0.5% |
| Triclosan | 0.3% |
| Disodium ethylenediaminetetraacetate | 0.5% |
| Perfume base | 0.5% |
| Purified water | Balance |

The body shampoo thus obtained had excellent detergency and foamability and also high deodorant effect.

### Example 11: (Antidandruffy shampoo)

An antidandruffy shampoo of a formulation shown below was produced in accordance with a method known *per se* in the art. -

| | |
|---|---|
| Disodium polyoxyethylene (2) lauryl | 12% |
| ether sulfosuccinate | |
| Lauroyldiethanolamide | 2% |
| Laurylhydroxysulfobetaine | 1% |
| Benzalkonium chloride | 0.5% |
| Zinc pyrithione | 0.2% |
| Disodium succinate | 0.4% |
| Perfume base | 0.5% |
| Purified water | Balance |

The antidandruffy shampoo thus obtained had excellent detergency and foamability and also high antidandruffy effect.

## Claims

1. A detergent composition comprising the following components (A), (B) and (C):
(A) 5-95 wt.% of an anionic surfactant;
(B) 0.2-5 wt.% of at least one germicide selected from the group consisting of cationic germicides represented by the following general formulae (2) : 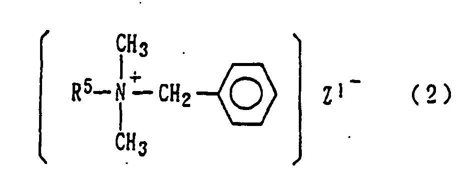 wherein R⁵ is a hydrocarbon group having 8-14 carbon atoms or a group represented by the formula: 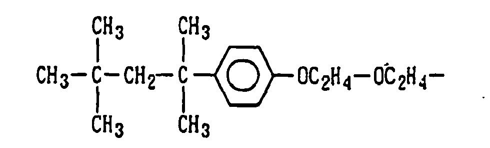 Z¹ is a halogen atom, an anionic residue of an amino acid, fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin;
and
(C) at least one antibacterial agent selected from the group consisting of triclosan, triclocarban, DMDM hydantoin, piroctone olamine, zinc pyrithione, selenium disulfide, climbazole and 3-methyl-4-(1-methylethyl)phenol, wherein the weight ratio (B)/(C) of the cationic germicide of the component (B) to the antibacterial agent of the component (C) is 0.1 to 25.

2. The detergent composition according to Claim 1, wherein the component (A) is at least one selected from the group consisting of higher fatty acid salts, polyoxyalkylene alkyl ether carboxylic acids and salts thereof, alkylsulfates, alkylsulfonates, alkylbenzenesulfonates, polyoxyalkylene alkyl ether sulfates, alkylphosphates, polyoxyalkylene alkyl ether phosphates, polyoxyalkylene alkylamide ether carboxylic acids and salts thereof, alkylsulfosuccinates, polyoxyalkylene alkyl ether sulfosuccinates, N-acyl-N-methyltaurinic acid salts, N-acylsarcosinates, α-olefin-sulfonates, acylated isethionates, and acylated glutamic acid and salts thereof.

3. The detergent composition according to Claim 1, wherein the component (A) is at least one selected from the group consisting of higher fatty acid salts, alkylsulfates, polyoxyalkylene alkyl ether sulfates, alkylphosphates, polyoxyalkylene alkylamide ether carboxylates, acylated isethionates, polyoxyalkylene alkyl ether carboxylic acids and polyoxyalkylene alkyl ether sulfosuccinates.

4. The detergent composition according to Claim 1, wherein the component (A) is at least one selected from the group consisting of alkylsulfates or polyoxyalkylene alkyl ether sulfates represented by the following general formula (5):
R⁷O-(CH₂CH₂O)ₙ-SO₃M¹ (5)
wherein R⁷ is a linear or branched alkyl or alkenyl group having 8-20 carbon atoms, n is a number of 0-10 on the average, and M¹ is an alkali metal or alkaline earth metal atom, or an ammonium, alkylammonium or alkanolammonium group; and higher fatty acid salts represented by the
following general formula (6):
R⁸COOX (6)
wherein R⁸ is a linear or branched alkyl or alkenyl group having 7-21 carbon atoms, and X is a potassium or sodium atom, or an ammonium or alkanolammonium group.

5. The detergent composition according to any one of Claims 1 to 4, wherein the component (B) is selected from the group consisting of benzalkonium chloride, and benzethonium chloride;

6. The detergent composition according to any one of Claims 1 to 5, wherein the component (C) is at least one selected from the group consisting of triclosan, triclocarban, piroctone olamine, DMDM hydantoin, zinc pyrithione, selenium disulfide and 3-methyl-4-(1-methylethyl)phenol.

7. The detergent composition according to any one of Claims 1 to 6, which further comprises a metal chelating agent.

8. The detergent composition according to Claim 7, wherein the metal chelating agent is selected from the group consisting of aminopolycarboxylic acid type chelating agents, aromatic and aliphatic carboxylic acid type chelating agents, amino acid type chelating agents, ether polycarboxylic acid type chelating agents, phosphonic acid type chelating agents, hydroxycarboxylic acid type chelating agents, phosphoric acid type chelating agents, chelating agents of the polyelectrolyte (including oligomer electrolyte) type, and dimethylglyoxime.

## Patentansprüche

1. Detergenszusammensetzung, umfassend die folgenden Komponenten (A), (B) und (C):
(A) 5 bis 95 Gew.% eines anionischen Tensids;
(B) 0,2 bis 5 Gew.% von mindestens einem Germicid, ausgewählt aus der Gruppe bestehend aus kationischen Germiciden, dargestellt durch die folgende Formel (2): 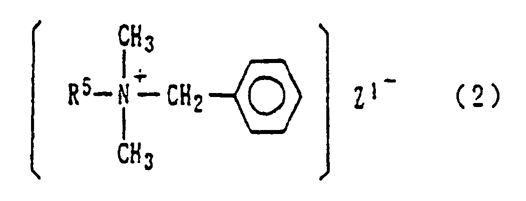 worin R⁵ eine Kohlenwasserstoffgruppe mit 8 bis 14 Kohlenstoffatomen oder eine durch die folgende Formel dargestellte Gruppe ist: 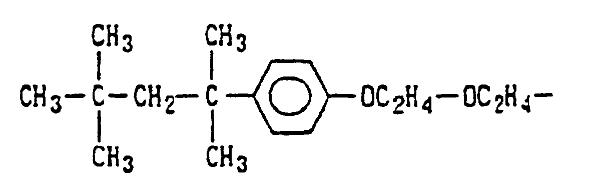 Z¹ ein Halogenatom, ein anionischer Rest einer Aminosäure, einer Fettsäure oder eines Phosphats, Phosphonats, Sulfonats oder Sulfats mit einer linearen oder verzweigten Alkyl- oder Alkenylgruppe mit 1 bis 30 Kohlenstoffatomen, oder ein anionisches Oligomer oder Polymer, das eine Styrolsulfonsäure mit einem Polymerisationsgrad von mindestens 3 aufweist, oder das ein Kondensat einer sulfonierten polycyclischen aromatischen Verbindung, die als eine Substituentengruppe eine Kohlenwasserstoffgruppe aufweisen kann, mit Formalin enthält; und
(C) mindestens ein antibakterielles Mittel, ausgewählt aus der Gruppe bestehend aus Triclosan, Triclocarban, DMDM-Hydantoin, Piroctonolamin, Zinkpyrithion, Selendisulfid, Climbazol und 3-Methyl-4-(1-methylethyl)phenol, worin das Gewichtsverhältnis (B)/(C) des kationischen Germicids der Komponente (B) zu dem antibakteriellen Mittel der Komponente (C) 0,1 bis 25 beträgt.

2. Detergenszusammensetzung gemäß Anspruch 1, worin die Komponente (A) mindestens eine ist, ausgewählt aus der Gruppe bestehend aus höheren Fettsäuresalzen, Polyoxyalkylenalkylether-Carbonsäuren und Salzen hiervon, Alkylsulfaten, Alkylsulfonaten, Alkylbenzolsulfonaten, Polyoxyalkylenalkylethersulfaten, Alkylphosphaten, Polyoxyalkylenalkyletherphosphaten, Polyoxyalkylenalkylamidether-Carbonsäuren und Salzen hiervon, Alkylsulfosuccinaten, Polyoxyalkylenalkylethersulfosuccinaten, N-Acyl-N-methyltaurinsäuresalzen, N-Acylsarcosinaten, α-Olefinsulfonaten, acylierten Isothionaten und acylierten Glutamsäuren und Salzen hiervon.

3. Detergenszusammensetzung gemäß Anspruch 1, worin die Komponente (A) mindestens eine ist, ausgewählt aus der Gruppe bestehend aus höheren Fettsäuresalzen, Alkylsulfaten, Polyoxyalkylenalkylethersulfaten, Alkylphosphaten, Polyoxyalkylenalkylamidethercarboxylaten, acylierten Isothionaten, Polyoxyalkylenalkylether-Carbonsäuren und Polyoxyalkylenalkylethersulfosuccinaten.

4. Detergenszusammensetzung gemäß Anspruch 1, worin die Komponente (A) mindestens eine ist, ausgewählt aus der Gruppe bestehend aus Alkylsulfaten oder Polyoxyalkylenalkylethersulfaten, dargestellt durch die folgende allgemeine Formel (5):
R⁷O-(CH₂CH₂O)ₙ-SO₃M¹ (5)
worin R⁷ eine lineare oder verzweigte Alkyl- oder Alkenylgruppe mit 8 bis 20 Kohlenstoffatomen ist, n durchschnittliche eine Zahl von 0 bis 10 ist und M¹ ein Alkalimetall oder Erdalkalimetall oder eine Ammonium-, Alkylammonium- oder Alkanolammoniumgruppe ist; und höheren Fettsäuresalzen, dargestellt durch die folgende allgemeine Formel (6):
R⁸COOX (6)
worin R⁸ eine lineare oder verzweigte Alkyl- oder Alkenylgruppe mit 7 bis 21 Kohlenstoffatomen ist und X Kalium oder Natrium oder eine Ammonium- oder Alkanolammoniumgruppe ist.

5. Detergenszusammensetzung gemäß irgendeinem der Ansprüche 1 bis 4, worin die Komponente (B) ausgewählt ist aus der Gruppe bestehend aus Benzalkoniumchlorid und Benzethoniumchlorid.

6. Detergenszusammensetzung gemäß irgendeinem der Ansprüche 1 bis 5, worin die Komponente (C) mindestens eine ist, ausgewählt aus der Gruppe bestehend aus Triclosan, Triclocarban, Piroctonolamin, DMDM-Hydantoin, Zinkpyrithion, Selendisulfid und 3-Methyl-4-(1-methylethyl)phenol.

7. Detergenszusammensetzung gemäß irgendeinem der Ansprüche 1 bis 6, die ferner ein Metall-chelatierendes Mittel umfasst.

8. Detergenszusammensetzung gemäß Anspruch 7, worin das Metall-chelatierende Mittel ausgewählt ist aus der Gruppe bestehend aus Chelat-bildenden Verbindungen vom Aminopolycarbonsäuretyp, Chelat-bildenden Mitteln vom aromatischen und aliphatischen Carbonsäuretyp, Chelat-bildenden Mitteln vom Aminosäuretyp, Chelat-bildenden Mitteln vom Ether-Polycarbonsäuretyp, Chelat-bildenden vom Phosphonsäuretyp, Chelat-bildenden Mitteln vom Hydroxycarbonsäuretyp, Chelat-bildenden Mitteln vom Phosphorsäuretyp, Chelat-bildenden Mitteln vom Polyelektrolyt (incl. Oligomer-Elektrolyt)-Typ und Dimethylglyoxim.

## Revendications

1. Composition détergente comprenant les composants (A), (B) et (C) suivants :
(A) 5-95 % en poids d'un tensioactif anionique ;
(B) 0,2-5 % en poids d'au moins un germicide choisi dans le groupe consistant en les germicides cationiques représentés par la formule générale (2) suivante : 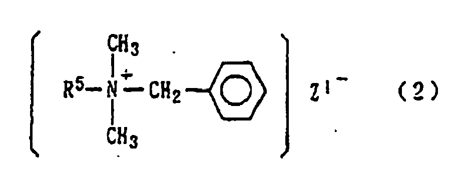 où R⁵ est un groupe hydrocarboné ayant 8-14 atomes de carbone ou un groupe représenté par la formule : 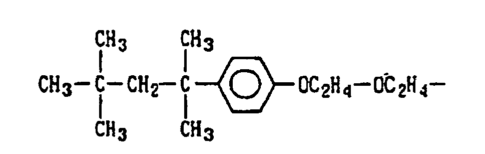 Z¹ est un atome d'halogène, un résidu anionique d'un aminoacide, d'un acide gras, ou un phosphate, phosphonate, sulfonate ou sulfate ayant un groupe alkyle ou alcényle linéaire ou ramifié ayant 1-30 atomes de carbone, ou un oligomère ou polymère anionique ayant un acide styrènesulfonique ayant un degré de polymérisation d'au moins 3 ou contenant un condensat d'un composé aromatique polycyclique sulfoné, qui peut avoir un groupe hydrocarboné comme groupe substituant, avec le formaldéhyde ;
(C) au moins un agent antibactérien choisi dans le groupe consistant en le triclosan, le triclocarban, la DMDM hydantoïne, la piroctone olamine, la pyrithione de zinc, le disulfure de sélénium, le climbazole et le 3-méthyl-4-(1-méthyléthyl)phénol, où le rapport en poids (B)/(C) du germicide cationique du composant (B) à l'agent antibactérien du composant (C) est 0,1 à 25.

2. Composition détergente selon la revendication 1 où le composant (A) est au moins un choisi dans le groupe consistant en les sels d'acides gras supérieurs, les acides polyoxyalkylène alkyl éther carboxyliques et leurs sels, les alkylsulfates, les alkylsulfonates, les alkylbenzènesulfonates, les polyoxyalkylène alkyl éther sulfates, les alkylphosphates, les polyoxyalkylène alkyl éther phosphates, les acides polyoxyalkylène alkylamide éther carboxyliques et leurs sels, les alkylsulfosuccinates, les polyoxyalkylène alkyl éther sulfosuccinates, les sels d'acides N-acyl-N-méthyltauriniques, les N-acylsarcosinates, les α-oléfine-sulfonates, les iséthionates acylés, et l'acide glutamique acylé et ses sels.

3. Composition détergente selon la revendication 1 où le composant (A) est au moins un choisi dans le groupe consistant en les sels d'acides gras supérieurs, les alkylsulfates, les polyoxyalkylène alkyl éther sulfates, les alkylphosphates, les polyoxyalkylène alkylamide éther carboxylates, les iséthionates acylés, les acides polyoxyalkylène alkyl éther carboxyliques et les polyoxyalkylène alkyl éther sulfosuccinates.

4. Composition détergente selon la revendication 1 où le composant (A) est au moins un choisi dans le groupe consistant en les alkylsulfates ou les polyoxyalkylène alkyl éther sulfates représentés par la formule générale (5) suivante :
R⁷O-(CH₂CH₂O)ₙ-SO₃M' (5)
où R⁷ est un groupe alkyle ou alcényle linéaire ou ramifié ayant 8-20 atomes de carbone, n est un nombre de 0-10 en moyenne, et M¹ est un atome de métal alcalin ou de métal alcalino-terreux, ou un groupe ammonium, alkylammonium ou alcanolammonium ; et les sels d'acides gras supérieurs représentés par la formule générale (6) suivante :
R⁸COOX (6)
où R⁸ est un groupe alkyle ou alcényle linéaire ou ramifié ayant 7-21 atomes de carbone, et X est un atome de potassium ou de sodium, ou un groupe ammonium ou alcanolammonium.

5. Composition détergente selon l'une quelconque des revendications 1 à 4 où le composant (B) est choisi dans le groupe consistant en le chlorure de benzalkonium et le chlorure de benzéthonium.

6. Composition détergente selon l'une quelconque des revendications 1 à 5 où le composant (C) est au moins un choisi dans le groupe consistant en triclosan, triclocarban, piroctone olamine, DMDM hydantoïne, pyrithione de zinc, disulfure de sélénium et 3-méthyl-4-(1-méthyléthyl)phénol.

7. Composition détergente selon l'une quelconque des revendications 1 à 6 qui comprend en outre un agent chélatant les métaux.

8. Composition détergente selon la revendication 7 où l'agent chélatant les métaux est choisi dans le groupe consistant en les agents chélatants de type acide aminopolycarboxylique, les agents chélatants de type acide carboxylique aromatique et aliphatique, les agents chélatants de type aminoacide, les agents chélatants de type acide éther polycarboxylique, les agents chélatants de type acide phosphonique, les agents chélatants de type acide hydroxycarboxylique, les agents chélatants de type acide phosphorique, les agents chélatants du type polyélectrolyte (incluant un électrolyte oligomère), et le diméthylglyoxime.
